# EUROPEAN PATENT SPECIFICATION

(11) **EP 3 564 219 B1**
(45) Date of publication and mention of the grant of the patent: **24.02.2021**
(21) Application number: 16925048.7
(22) Date of filing: 30.12.2016
(51) Int. Cl.: C07D 273/06

(54) **PROCESS FOR PREPARING OXADIAZACYCLO COMPOUND AND USE THEREOF**
VERFAHREN ZUR HERSTELLUNG EINER OXADIAZACYCLO-VERBINDUNG UND DEREN VERWENDUNG
PROCÉDÉ DE PRÉPARATION D'UN COMPOSÉ OXADIAZACYCLO ET SON UTILISATION

(43) Date of publication of application: 06.11.2019
(73) Proprietor: Oriental (Luzhou) Agrochemicals. Co., Ltd., Luzhou City, Sichuan 646300 (CN)
(72) Inventor: SUN, Yinwei, Hangzhou Zhejiang 310052 (CN); LAI, Teng, Hangzhou Zhejiang 310052 (CN); ZHANG, Pan, Hangzhou Zhejiang 310052 (CN); CHEN, Bangchi, Hangzhou Zhejiang 310052 (CN)
(74) Representative: Haseltine Lake Kempner LLP
(86) International application number: PCT/CN2016/113669
(87) International publication number: WO 2018/120093

(56) References cited:
- WO-A1-99/47525
- WO-A1-2006/045587
- CN-A- 1 292 791
- CN-A- 1 604 896
- CN-A- 101 039 926

## Description

### TECHNICAL FIELD

The present invention relates to synthesis of organic oxazacyclic compounds, and specifically to a method for preparing an oxadiazacyclic compound and uses thereof.

### BACKGROUND OF THE INVENTION

Organic oxazacyclic compounds are an important class of organic heterocyclic compounds. For example, [1,4,5]-oxadiazepine is an important intermediate for synthesizing herbicide Pinoxaden (WO 99047525).

WO 99047525 disclosed a method for synthesizing [1,4,5]-oxadiazepine hydrobromide in which N,N'-di-tert-butoxycarbonyl hydrazine is cyclized with 2,2'-dimethylsulfonyloxydiethyl ether in the presence of a base to produce N,N'-di-tert-butoxycarbonyl-[1,4,5]-oxadiazepine which is then reacted with hydrobromic acid in ethyl ether to give [1,4,5]-oxadiazepine hydrobromide. Despite of a relatively high yield, this method involves the use of BoC₂O that is expensive and has a large molecular weight for introducing protective groups, resulting in a large amount of waste during deprotection. In addition, the hydrobromic acid used is very corrosive to the equipment, and ethyl ether used as the solvent has a low flash point causing safety problems. Moreover, this method also involves a long reaction time (48 h) and low efficiency, and the product [1,4,5]-oxadiazepine hydrobromide is easily moistened and poor in thermal stability.

WO 02051853 disclosed a method for synthesizing [1,4,5]-oxadiazepine hydrohalide in which N,N'-diacyl hydrazine is cyclized with a disubstituted ether in the presence of an inorganic base to produce N,N'-diacyl-[1,4,5]-oxadiazepine which is then reacted with a halogen acid to give [1,4,5]-oxadiazepine hydrohalide. Although this method solves the problems caused by usage of BoC₂O, which is high cost, large molecular weight of protection groups, much wastes during deprotection process. All other defects are still existing, including not only long reaction time, high usage rate of solvent, but also those from the product [1,4,5]-oxadiazepine hydrobromide such as easy absorption of moisture, thermal instability and prominent corrosion to equipment (WO 2006045587). In addition, the yield of such synthetic process to produce N,N'-diacyl- [1,4,5]-oxadiazepine is low, which is 76% at most.

Base on WO02051853, WO2006045587 disclosed a method for synthesizing [1,4,5]-oxadiazepine by reacting N,N'-diacyl-[1,4,5]-oxadiazepine with an inorganic base such as potassium hydroxide in a polar solvent. This method solves the above problems caused by the halogen acid. However, a higher yield (65-90%) is possible only when water is used as the solvent. Meanwhile, a large amount of solid waste is formed due to the use of organic salts. Furthermore, it requires multiple extraction operation, because the [1,4,5]-oxadiazepine is difficult to separate due to its extremely high water-solubility, leading to an increased cost and production of waste liquor. This method is still limited by the low yield of N,N'-diacyl-[1,4,5]-oxadiazepine and low total yield (49%-69%).

To solve the above problems, the inventors of the present invention, through numerous researches and experiments, have surprisingly found that employing N-alkoxyacyl-N'-acyl hydrazine as a raw material can not only solve the defects of low yield of cyclization reaction of the acyl group-protected substrates, but also the problems caused by [1,4,5]-oxadiazepine hydrobromide, such as easy absorption of moisture, thermal instability, corrosion to equipment and so on.

Given the fact that the inorganic base such as potassium hydroxide has an extremely low solubility in the non-polar solvent such toluene and xylene, those skilled in the art will not perform the hydrolysis reaction of amide compounds using such a combination of inorganic base and non-polar solvent. Nevertheless, the inventors of the present invention, through numerous researches and experiments, have surprisingly found that N-alkoxyacyl-N'-acyloxadiazacyclic compounds can directly react with a base in a non-polar solvent, moreover, the yield is extremely high.

### SUMMARY OF THE INVENTION

The invention provides a novel method for preparing an oxadiazacyclic compound, comprising:
step 1: cyclizing N-alkoxycarbonyl hydrazine (1) with a disubstituted ether (2) in the presence of a base to produce a N-alkoxycarbonyl oxadiazacyclic compound (3); and
step 2: reacting compound (3) with a base to produce oxadiazacyclic compound (4);
   as shown in the following reaction scheme: 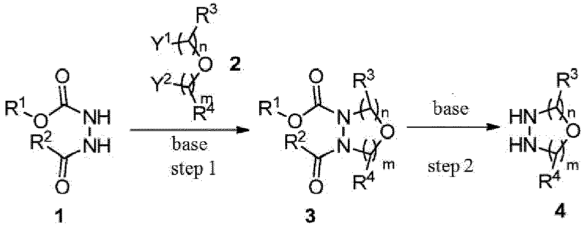 wherein:
   R¹ is hydrogen, a C₁-C₆ alkyl group, a C₆-C₁₂ aryl group, or a heteroaryl group containing one or two atoms selected from nitrogen, oxygen and sulfur;
   R², R³ and R⁴ each are independently hydrogen, a C₁-C₆ alkyl group, a C₁-C₆ alkoxy group, a C₆-C₁₂ aryl group, or a heteroaryl group containing one or two atoms selected from nitrogen, oxygen and sulfur;
   Y¹ and Y² each are independently a halogen, a C₁-C₆ alkylsulfonyloxy group, or a C₆-C₁₂ arylsulfonyloxy group; and
   m and n each are independently 1 or 2.

In the cyclization of step 1, the base is selected from an alkali metal or alkaline earth metal hydroxide, carbonate, hydrogencarbonate, alcoholate, hydride and alkylate, or a mixture thereof; a molar ratio of the base to compound (1) is 1.5-4.0:1, preferably 2.0-2.2:1; a reaction solvent is selected from an aromatic hydrocarbon, an ether, an amide, or a mixture thereof, preferably an amide such as N,N-dimethylformamide; and a reaction temperature is -10-100°C, preferably 0-40°C.

In step 2, the base is selected from an alkali metal or alkaline earth metal hydroxide, carbonate and hydrogencarbonate, or a mixture thereof; a molar ratio of the base to compound (3) is 3.0-4.5:1, preferably 3.4-4.5:1; a reaction solvent is an aromatic hydrocarbon such as toluene, and is preferably an aromatic hydrocarbon solvent such as toluene or xylene; and a reaction temperature is 0-200°C, preferably 100-130°C.

Step(s) 1 and/or 2 may be performed in the presence of a phase transfer catalyst, wherein the phase transfer catalyst is selected from a quaternary ammonium salt, a quaternary phosphonium salt, and cyclic crown ethers such as 18-crown-6, and is preferably a quaternary ammonium salt, more preferably tetrabutylammonium bromide.

In the present invention, the solution of [1,4,5]-oxadiazepine in a non-polar solvent can be directly used for the preparation of drugs such as herbicide Pinoxaden without purification, enabling further simplified entire process and reduced wastes and cost, and benefiting the industrial production.

The method of preparing oxadiazaclic compounds afforded by the present invention employs a novel N-protection strategy, avoiding the use of expensive protective agent Boc₂O. Meanwhile, the method can undergo cyclization reaction more efficiently, compared with method of N-acyl group protection, improving reaction yield and avoiding corrosion to equipment. Furthermore, the method, compared to the prior art, has particularly the following advantages: (1) the product yield of the present invention is generally higher than 90%; (2) excessive inorganic salts are not required, reducing the production of waste; (3) the post-treatment is simple, specifically, the byproduct salts can be easily removed by filtration due to their insolubility in the non-polar solvent; (4) the non-polar solvent used herein eliminates the need for separation of the product through aqueous two-phase extraction, thereby reducing the liquid waste; (5)the solution of the resulting product in the non-polar solvent can be directly used for the synthesis of herbicides such as Pinoxaden without purifying process, enabling further simplified entire process and reduced wastes and cost, and benefiting the industrial production.

### DETAILED DESCRIPTION OF EMBODIMENTS

The features of the present invention will be further illustrated with reference to the embodiments, but the embodiments are not intended to limit the scope of the invention.

### Preparation of raw materials

### Preparation of N-methoxycarbonyl-N'-isopropoxycarbonyl hydrazine

A solution of 90.1 g of N-methoxycarbonyl hydrazine (1.00 mol) in methanol was dropwise added to a solution of 55.1 g of sodium methylate (1.02 mol) in methanol under nitrogen protection to react for 0.5 h. Then 122.5 g of isopropyl chloroformate (1.00mol) was slowly dropwise added to the reaction mixture. After addition, the reaction mixture reacted at 65°C for 6 h to give a pale-yellow cloudy liquid. The solvent was evaporated, and the remaining reaction mixture was adjusted to pH of 6 with 10% hydrochloric acid, extracted with ethyl acetate. The organic phase was dried and concentrated to give a crude product. The crude product was recrystallized with ethyl acetate/petroleum ether to give 105.7g of N-methoxycarbonyl-N'-isopropoxycarbonyl hydrazine as a white solid, and the yield was 60%.
¹H NMR (CDCl₃, 500MHz, TMS): δ 6.96 (brs, 1H), 6.77 (brs, 1H), 5.01-4.94 (m, 1H), 3.76 (s, 3H), 1.26 (d, J = 6.5 Hz, 6H).
¹³C NMR (CDCl₃, 125 MHz): δ 156.5, 71.2, 52.2, 21.3.

### Preparation of N-methoxycarbonyl-N'-formyl hydrazine

To a 500 mL reaction flask were sequentially added 90.1 g of N-methoxycarbonyl hydrazine (1.00 mol), 24.4 g of 4-dimethylaminopyridine (0.20 mol) and 66.7 g of ethyl formate (0.90 mol) under nitrogen protection. The reaction mixture was heated and refluxed for 6 h. Then the reaction mixture was adjusted to pH of 6 with 10 % hydrochloric acid, extracted with ethyl acetate. The organic phase was dried and concentrated to give a crude product. The crude product was recrystallized with ethyl acetate/petroleum ether to give 75.5 g of N-methoxycarbonyl-N'-formyl hydrazine as a white solid, and the yield was 71%.
¹H NMR (CDCl₃, 500MHz, TMS): δ 9.68 (br, 1H), 8.64 (br, 1H), 8.13-8.11 (m, 1H), 3.76-3.68 (m, 3H).
¹³C NMR (CDCl₃, 125 MHz): δ 160.3, 156.7, 53.2.

### Preparation of N-methoxycarbonyl-N'-benzoyl hydrazine

A solution of 90.1 g of N-methoxycarbonyl hydrazine (1.00 mol) in methanol was dropwise added to a solution of 55.1 g of sodium methylate (1.02 mol) in methanol under nitrogen protection to react for 0.5 h. Then 140.6 g of benzoyl chloride (1.00 mol) was slowly dropwise added. After addition, the reaction mixture reacted at 65°C for 6 h to give a pale-yellow cloudy liquid. The solvent was evaporated, and the remaining reaction mixture was adjusted to pH of 6 with 10 % hydrochloric acid, extracted with ethyl acetate. The organic phase was dried and subjected to rotary evaporation to remove the solvent to give a crude product. The crude product was recrystallized with ethyl acetate/petroleum ether to give 153.4 g of N-methoxycarbonyl-N'-benzoyl hydrazine as a white solid, and the yield was 79%.
¹H NMR (CDCl₃, 500MHz, TMS): δ 8.52 (brs, 1H), 7.83-7.81 (m, 2H), 7.54-7.51 (m, 1H), 7.44-7.40 (m, 2H), 7.20 (brs, 1H), 3.73 (s, 3H).
¹³C NMR (CDCl₃, 125 MHz): δ 167.1, 157.3, 132.4, 131.5, 128.7, 127.3, 53.2.

### Preparation of N-methoxycarbonyl-N'-acetyl hydrazine

A solution of 90.1 g of N-methoxycarbonyl hydrazine (1.00 mol) in methanol was dropwise added to a solution of 55.1 g of sodium methylate (1.02 mol) in methanol under nitrogen protection to react for 0.5 h. Then 78.5 g of acetyl chloride (1.00 mol) was slowly dropwise added. After addition, the reaction mixture reacted at 65°C for 6 h to give a pale-yellow cloudy liquid. The solvent was evaporated, and the remaining reaction mixture was adjusted to pH of 6 with 10% hydrochloric acid, extracted with ethyl acetate. The organic phase was dried and subjected to rotary evaporation to remove the solvent to give a crude product. The crude product was recrystallized with ethyl acetate/petroleum ether to give 113.6 g of N-methoxycarbonyl-N'-acetyl hydrazine as a white solid, and the yield was 86%.
¹H NMR (CDCl₃, 500 MHz, TMS): δ 8.99 (br, 1H), 8.01 (br, 1H), 3.73 (s, 3H), 2.02 (s, 3H).
¹³C NMR (CDCl₃, 125 MHz): δ 170.8, 157.4, 52.9, 20.3.

### Example 1 Preparation of N-methoxycarbonyl-N'-isopropoxycarbonyl-[1,4,5] -oxadiazepine

A solution of 70.4 g of N-methoxycarbonyl-N'-isopropoxycarbonyl hydrazine (0.40 mol) in N,N-dimethylformamide was dropwise added to a suspension of 32.0 g of sodium hydride (0.80 mol) in N,N-dimethylformamide at a low temperature. After addition, the reaction mixture reacted under elevated temperature to remove the hydrogen formed during reaction. Then the reaction mixture was cooled to 0-5°C. A solution of 104.9 g of 2,2'-dimethylsulfonyl diethyl ether (0.40 mol) in N, N-dimethylformamide was dropwise added. Then the reaction mixture reacted at room temperature. After the reaction was complete, the reaction was quenched, and the reaction mixture was extracted with methyl tert-butyl ether. The organic phases were combined, washed with water, dried and concentrated to give 89.6 g of N-methoxycarbonyl-N'- isopropoxycarbonyl- [1,4,5]-oxadiazepine as a colorless oily liquid, and the yield was 91%.
¹H NMR (CDCl₃, 500MHz, TMS): δ 4.99-4.94 (m, 1H), 4.15-4.11 (m, 1H), 4.03-4.01 (m, 1H), 3.87-3.63 (m, 7H), 3.38- 3.22 (m, 2H), 1.30-1.21 (m, 6H).
¹³C NMR (CDCl₃, 125MHz): δ 155.88, 154.85, 69.7, 69.2, 69.0, 53.2, 50.5, 21.9, 21.8.

### Example 2 Preparation of N,N'-dimethoxycarbonyl[1,4,5]-oxadiazepine

500 mL of a solution of 148.1 g of N,N'-dimethoxycarbonyl hydrazine (1.00 mol) in N,N-dimethylformamide was dropwise added to a suspension of 84.0 g of sodium hydride (2.10 mol) in N,N-dimethylformamide at a low temperature. After addition, the reaction mixture reacted under elevated temperature to remove the hydrogen formed during reaction. Then the reaction mixture was cooled to 0-5°C. A solution of 275.4 g of 2,2'-dimethylsulfonyl diethyl ether (1.05 mol) in N, N-dimethylformamide was dropwise added. The reaction mixture reacted at a temperature of 0-5°C until GC indicated that the raw materials were exhausted. The reaction was quenched, and then the reaction mixture was extracted with methyl tert-butyl ether. The organic phases were combined, washed with water, dried and concentrated to give 197.3 g of N, N'-dimethoxycarbonyl- [1,4,5]-oxadiazepine as a colorless oily liquid, the yield was 90%.
¹H NMR (CDCl₃, 500MHz, TMS): δ 4.18-4.15 (m, 1H), 4.04-4.00 (m, 1H), 3.89-3.75 (m, 8H), 3.70-3.66 (m, 2H), 3.36-3.25 (m, 2H).
¹³C NMR (CDCl₃, 125 MHz): δ 174.8, 174.1, 64.0, 63.5, 52.9, 52.7, 51.0, 50.6.

### Example 3 Preparation of N-methoxycarbonyl-N'-benzoyl-[1,4,5]-oxadiazepine

A solution of 97.1 g of N-methoxycarbonyl-N'-benzoyl hydrazine (0.50 mol) in N,N-dimethylacetamide was dropwise added to a suspension of 42.0 g of sodium hydride (1.05 mol) in N,N-dimethylformamide at a low temperature. After addition, the reaction mixture reacted under elevated temperature to remove the hydrogen formed during reaction. Then the reaction mixture was cooled to 0-5°C. A solution of 74.4 g of 2,2'-dichlorodiethyl ether (0.52 mol) in N,N-dimethylacetamide was dropwise added. The reaction mixture reacted at room temperature. After the reaction was complete, the reaction was quenched, and the reaction mixture was extracted with methyl tert-butyl ether. The organic phases were combined, washed with water, dried and concentrated to give 125.4 g of N-methoxycarbonyl-N'-benzoyl-[1,4,5]-oxadiazepine as a colorless oily liquid, and the yield was 95%.
¹H NMR (CDCl₃, 500MHz, TMS): δ 7.46-7.35 (m, 5H), 4.51-4.37 (m, 1H), 4.08-3.93 (m, 1H), 3.89-3.64 (m, 9H), 3.52-3.04 (m, 2H).
¹³C NMR (CDCl₃, 125MHz): δ 172.6, 155.6, 130.2, 128.2, 128.1, 126.1, 68.9, 68.6, 68.4, 53.6, 52.0, 50.3.

### Example 4 Preparation of N-methoxycarbonyl-N'-acetyl-[1,4,5]-oxadiazepine

A solution of 66.0 g of N-methoxycarbonyl-N'-acetyl hydrazine (0.80 mol) in N,N-dimethylformamide was dropwise added to a suspension of 67.2 g of sodium hydride (1.68 mol) in N,N-dimethylformamide at a low temperature. After addition, the reaction mixture reacted under elevated temperature to remove the hydrogen formed during reaction. The reaction mixture was cooled to 0-5°C. A solution of 220.3 g of 2,2'-dimethylsulfonyl diethyl ether (0.83 mol) in N,N-dimethylformamide was dropwise added. The reaction mixture reacted at room temperature. After the reaction was complete, the reaction was quenched, and the reaction mixture was extracted with methyl tert-butyl ether. The organic phases were combined, washed with water, dried and concentrated to give 156.8 g of N-methoxycarbonyl-N'-acetyl-[1,4,5]-oxadiazepine as a colorless oily liquid, and the yield was 97%.
¹H NMR (CDCl₃, 500MHz, TMS): δ 4.38-4.26 (m, 1H), 4.18-4.11 (m, 1H), 4.05-3.91 (m, 2H), 3.88-3.67 (m, 5H), 3.35- 3.11 (m, 2H), 2.10-2.05 (m, 3H).
¹³C NMR (CDCl₃, 125 MHz): δ 170.0, 157.1, 70.3, 69.8, 68.9, 53.3, 50.6, 20.4.

### Example 5 Preparation of N-methoxycarbonyl-N'-formyl-[1,4,5]-oxadiazepine

A solution of 70.8 g of N-methoxycarbonyl-N'-formyl hydrazine (0.60 mol) in N,N-dimethylformamide was dropwise added to a suspension of 50.4 g of sodium hydride (1.26 mol) in N,N-dimethylformamide at a low temperature. After addition, the reaction mixture reacted under elevated temperature to remove the hydrogen formed during reaction. Then the reaction mixture was cooled to 0-5°C. A solution of 165.2 g of 2,2'-dimethylsulfonyl diethyl ether (0.62 mol) in N,N-dimethylformamide was dropwise added. The reaction mixture reacted at room temperature. After the reaction was complete, the reaction was quenched, and the reaction mixture was extracted with methyl tert-butyl ether. The organic phases were combined, washed with water, dried and concentrated to give 84.7 g of N-methoxycarbonyl-N'-formyl-[1,4,5]-oxadiazepine, and the yield was 75%.
¹H NMR (CDCl₃, 500MHz, TMS): δ 8.20 (s, 1H), 4.24-4.20 (m, 2H), 3.87-3.64 (m, 7H), 3.38-3.04 (m, 2H).
¹³C NMR (CDCl₃, 125 MHz): δ 164.5, 160.7, 68.9, 68.2, 53.9, 52.9, 47.8.

### Example 6 Preparation of [1,4,5]-oxadiazepine

109.0 g of N,N'-dimethoxycarbonyl-[1,4,5]-oxadiazepine (0.50 mol) was added to toluene. Then 126.2 g of potassium hydroxide (2.25 mol) was added. The reaction mixture was refluxed for 3 h. After the reaction was complete, the reaction mixture was cooled to room temperature and filtered to give a solution of [1,4,5]-oxadiazepine in toluene containing 50.0 g of product by GC analysis (98% yield).

### Example 7 Preparation of [1,4,5]-oxadiazepine

21.8 g of N,N'-dimethoxycarbonyl-[1,4,5]-oxadiazepine (0.10 mol) was added to xylene. Then 1.1 g of tetrabutyl ammonium bromide (0.003 mol) and 18.0 g of sodium hydroxide (0.45 mol) were added. The reaction mixture was heated to 130°C to react for 3 h. After the reaction was complete, the reaction mixture was cooled to room temperature and filtered to give a solution of [1,4,5]-oxadiazepine in toluene containing 8.7 g of product (85% yield).

### Example 8 Preparation of [1,4,5]-oxadiazepine (reference)

109.0 g of N,N'-dimethoxycarbonyl[1,4,5]-oxadiazepine (0.50 mol) was added to water .Then 112.2 g of potassium hydroxide (2.00 mol) was added. The reaction mixture was refluxed for 3 h until the reaction was complete. Then the reaction mixture was cooled to room temperature and extracted with toluene to give a solution of [1,4,5]-oxadiazepine in toluene containing 41.8 g of product (82% yield).

### Example 9 Preparation of [1,4,5]-oxadiazepine (reference)

36.3 g of N,N'-dimethoxycarbonyl-[1,4,5]-oxadiazepine (0.15 mol) was added to ethylene glycol. Then 40.7 g of potassium hydroxide (0.67 mol) was added. The reaction mixture was heated to 130°C to react for 3 h. After the reaction was complete, the reaction mixture was cooled to room temperature to give a solution of [1,4,5]-oxadiazepine in ethylene glycol containing 9.2 g of product (60% yield).

### Example 10 Preparation of [1,4,5]-oxadiazepine

73.9 g of N-methoxycarbonyl-N'-isopropoxycarbonyl-[1,4,5]-oxadiazepine (0.30 mol) was added to xylene. Then 75.7 g of potassium hydroxide (1.35 mol) was added. The reaction mixture was heated to 130°C to react for 2 h. After the reaction was complete, the reaction mixture was cooled to room temperature and filtered to give a solution of [1,4,5]-oxadiazepine in xylene containing 28.8 g of product (94% yield).

### Example 11 Preparation of [1,4,5]-oxadiazepine

26.4 g of N-methoxycarbonyl-N'-benzoyl-[1,4,5]-oxadiazepine (0.10 mol) was added to xylene. Then 18.9 g of potassium hydroxide (0.34 mol) was added. The reaction mixture was heated to 130°C to react for 2 h. After the reaction was complete, the reaction mixture was cooled to room temperature and filtered to give a solution of [1,4,5]-oxadiazepine in xylene containing 9.9 g of product (97% yield).

### Example 12 Preparation of [1,4,5]-oxadiazepine

60.7 g of N-methoxycarbonyl-N'-acetyl-[1,4,5]-oxadiazepine (0.30 mol) was added to xylene. Then 57.2 g of potassium hydroxide (1.02 mol) was added. The reaction mixture was heated to 130°C to react for 2 h. After the reaction was complete, the reaction mixture was cooled to room temperature and filtered to give a solution of [1,4,5]-oxadiazepine in xylene containing 28.8 g of product (94% yield).

### Example 13 Preparation of [1,4,5]-oxadiazepine

18.8 g of N-methoxycarbonyl-N'-formyl-[1,4,5]-oxadiazepine (0.10 mol) was added to xylene. Then 18.9 g of potassium hydroxide (0.34 mol) was added. The reaction mixture was heated to 130°C to react for 2 h. After the reaction was complete, the reaction mixture was cooled to room temperature and filtered to give a solution of [1,4,5]-oxadiazepine in xylene containing 9.49 g of product (93% yield).

### Example 14 Preparation of [1,4,5]-oxadiazepine

87.3 g of N,N'-dimethoxycarbonyl-[1,4,5]-oxadiazepine (0.300 mol) was added to xylene. Then 4.4 g of tetrabutyl ammonium bromide (0.01 mol) and 75.7 g of potassium hydroxide (1.35 mol) were added. The reaction mixture was refluxed until the reaction was complete. Then the reaction mixture was filtered to give a solution of [1,4,5]-oxadiazepine in xylene containing 29.4 g of product (96% yield).

### Example 15 Preparation of N,N'-dimethoxycarbonyl-[1,4,5]-oxadiazepine

Solutions of 74.0 g of N, N'-dimethoxycarbonyl hydrazine (0.50 mol), 138.2 g of potassium carbonate (1.00 mol) and 74.4 g of 2,2'-dichlorodiethyl ether (0.52 mol) in N,N-dimethylformamide were sequentially added at a low temperature. Then the suspension reaction mixture was heated to 100°C for reaction. After the reaction was complete, the reaction mixture was extracted with methyl tert-butyl ether. The organic phases were combined, washed with water, dried and concentrated to give 92.7 g of N,N'-dimethoxycarbonyl-[1,4,5]-oxadiazepine as a colorless oily liquid, and the yield was 85%.

### Example 16 Preparation of N,N'-dimethoxycarbonyl-[1,4,5]-oxadiazepine

Solutions of 118.5 g of N, N'-dimethoxycarbonyl hydrazine (0.80 mol), 98.7 g of potassium hydroxide (1.76 mol) and 118.8 g of 2,2'-dichlorodiethyl ether (0.83 mol) in dimethyl sulfoxide were sequentially added at a low temperature. Then the suspension reaction mixture was heated to 100°C for reaction. After the reaction was complete, the reaction mixture was extracted with methyl tert-butyl ether. The organic phases were combined, washed with water, dried and concentrated to give 139.6 g of N,N'-dimethoxycarbonyl-[1,4,5]-oxadiazepine as a colorless oily liquid, and the yield was 80%.

### Example 17 Preparation of Pinoxaden

59.6 g of 2-(2,6-diethyl-4-methylphenyl) malonamide (0.24 mol) and 43.7 g of triethylamine (0.43 mol) were sequentially added to the solution of [1,4,5]-oxadiazepine in xylene prepared in Example 14. The reaction mixture was refluxed until the reaction was complete. Then the reaction mixture was cooled to room temperature. 52.1 g of pivaloyl chloride (0.43 mol) was added. The reaction mixture reacted at room temperature. After the reaction was complete, the reaction mixture was washed with 1 N hydrochloric acid, and then extracted with ethyl acetate. The organic phases were combined, dried and crystallized by concentration to give 68.5 g of Pinoxaden, and the yield was 71%.
¹H NMR (CDCl₃, 500MHz, TMS): δ 8.88 (s, 2H), 4.28-4.26 (m, 2H), 3.94-3.93 (m, 2H), 3.89-3.83 (m, 4H), 2.56-2.47 (m, 2H), 2.45-2.40 (m, 2H), 2.39 (s, 3H), 1.12 (t, J = 9.0 Hz, 3H), 1.23 (s, 9H).

## Claims

1. A method for preparing an oxadiazacyclic compound (4), comprising:
step 1: cyclizing compound (1) with compound (2) in the presence of a base to produce compound (3); and
step 2: reacting compound (3) with a base to produce the oxadiazacyclic compound (4);
as shown in the following reaction scheme: 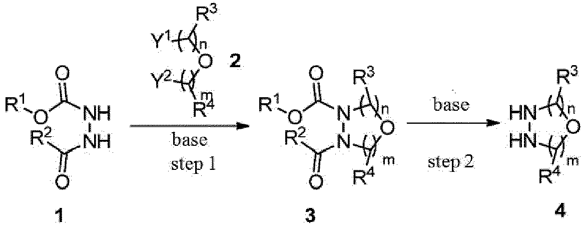 wherein in step 2
the base is selected from an alkali metal or alkaline earth metal hydroxide, carbonate and hydrogen-carbonate or a mixture thereof; a molar ratio of the base to compound (3) is 3.0-4.5:1, a reaction solvent is an aromatic hydrocarbon; and a reaction temperature is 0-200°C;
wherein:
R¹ is a C₁-C₆ alkyl group, a C₆-C₁₂ aryl group, or a heteroaryl group containing one or two atoms selected from nitrogen, oxygen and sulfur;
R² is hydrogen, a C₁-C₆ alkyl group, a C₆-C₁₂ aryl group, a heteroaryl group containing one or two atoms selected from nitrogen, oxygen and sulfur, a C₁-C₆ alkoxy group, or a C₆-C₁₂ aryloxy group;
R³ and R⁴ each are independently hydrogen, a C₁-C₆ alkyl group, a C₆-C₁₂ aryl group, or a heteroaryl group containing one or two atoms selected from nitrogen, oxygen and sulfur;
Y¹ and Y² each are independently a halogen, a C₁-C₆ alkylsulfonyloxy group, or a C₆-C₁₂ arylsulfonyloxy group; and
m and n each are independently 1 or 2.

2. The method according to claim 1, **characterized in that**:
R¹ is a C₁-C₃ alkyl group;
R² is selected from a C₁-C₃ alkyl group, a C₁-C₃ alkoxy group and a C₆-C₁₂ aryl group;
R³ and R⁴ each are independently hydrogen;
m and n each are independently 2;
in step 1, the base in the cyclization reaction is selected from an alkali metal or alkaline earth metal hydroxide, carbonate, hydrogencarbonate, alcoholate, hydride and alkylate, or a mixture thereof;
in step 1, a molar ratio of the base to compound (1) is 1.5-4.0:1, a reaction solvent is selected from an aromatic hydrocarbon, an ether, an amide, or a mixture thereof, and a reaction temperature is -10-150°C.

3. The method according to claim 2, **characterized in that**:
in step 1, the base in the cyclization reaction is sodium hydride, the molar ratio of the base to compound (1) is 2.0-2.2:1, the reaction solvent is N,N-dimethylformamide, and the reaction temperature is 0-100°C; and
in step 2, the base is potassium hydroxide, the molar ratio of the base to compound (3) is 3.4-4.5:1, the reaction solvent is toluene or xylene, and the reaction temperature is 100-130°C.

4. A method for preparing an oxadiazacyclic compound (4), comprising:
reacting compound (3) with a base to produce oxadiazacyclic compound (4), as shown in the following reaction scheme: 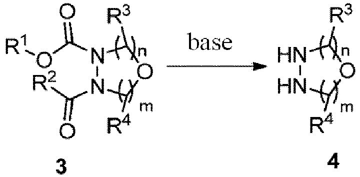 wherein:
R¹ is a C₁-C₆ alkyl group, a C₆-C₁₂ aryl group, or a heteroaryl group containing one or two atoms selected from nitrogen, oxygen and sulfur;
R² is hydrogen, a C₁-C₆ alkyl group, a C₆-C₁₂ aryl group, a heteroaryl group containing one or two atoms selected from nitrogen, oxygen and sulfur, a C₁-C₆ alkoxy group, or a C₆-C₁₂ aryloxy group;
R³ and R⁴ each are independently hydrogen, a C₁-C₆ alkyl group, a C₆-C₁₂ aryl group, or a heteroaryl group containing one or two atoms selected from nitrogen, oxygen and sulfur; and
m and n each are independently 1 or 2;wherein
the base is selected from an alkali metal or alkaline earth metal hydroxide, carbonate and hydrogencarbonate, or a mixture thereof;
a molar ratio of the base to compound (3) is 3.0-4.5:1;
a reaction solvent is an aromatic hydrocarbon; and
a reaction temperature is 0-200°C.

5. The method according to claim 4, **characterized in that**:
the base is potassium hydroxide;
the molar ratio of the base to compound (3) is 3.4-4.5:1;
the reaction solvent is toluene or xylene; and
the reaction temperature is 100-130°C.

6. The method according to any one of claims 1 to 5, **characterized in that** each step is performed in the presence of a phase transfer catalyst, wherein the phase transfer catalyst is selected from a quaternary ammonium salt, a quaternary phosphonium salt and a cyclic crown ether compound.

7. The method according to claim 6, **characterized in that** the phase transfer catalyst is preferably tetrabutylammonium bromide.

8. A compound of formula (3) 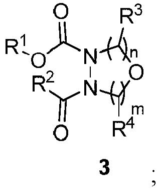 wherein:
R¹ is a C₁-C₆ alkyl group or a C₆-C₁₂ aryl group;
R² is hydrogen, a C₁-C₆ alkyl group, a C₆-C₁₂ aryl group, a heteroaryl group containing one or two atoms selected from nitrogen, oxygen and sulfur, a C₁-C₆ alkoxy group, or a C₆-C₁₂ aryloxy group, and R² is not a tert-butoxy group when R¹ is a tert-butyl group;
R³ and R⁴ each are independently hydrogen, a C₁-C₆ alkyl group, a C₆-C₁₂ aryl group, or a heteroaryl group containing one or two atoms selected from nitrogen, oxygen and sulfur; and
m and n each are independently 1 or 2.

9. The compound according to claim 8, **characterized in that**:
R¹ is a C₁-C₃ alkyl group;
R² is a C₁-C₃ alkyl group, a C₁-C₃ alkoxy group or a phenyl group;
R³ and R⁴ each are independently hydrogen; and
m and n each are independently 2.

## Patentansprüche

1. Verfahren zum Herstellen einer oxadiazacyclischen Verbindung (4), umfassend:
Schritt 1: Zyklisieren von Verbindung (1) mit Verbindung (2) in Anwesenheit einer Base, um Verbindung (3) herzustellen; und
Schritt 2: Reagieren der Verbindung (3) mit einer Base, um die oxadiazacyclische Verbindung (4) herzustellen;
wie es in dem folgenden Reaktionsschema gezeigt ist: 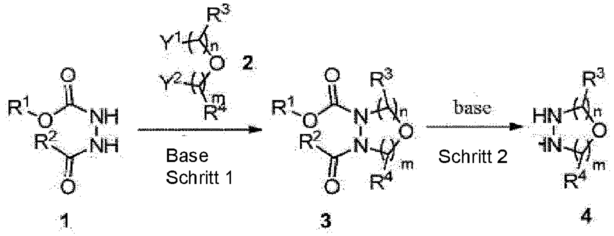 wobei in Schritt 2
die Base ausgewählt ist aus einem Alkalimetall- oder Erdalkalimetallhydroxid, Carbonat und Hydrogencarbonat oder einem Gemisch davon; ein molares Verhältnis der Base zu Verbindung (3) 3,0-4,5:1 ist, ein Reaktionslösungsmittel ein aromatischer Kohlenwasserstoff ist; und eine Reaktionstemperatur von 0-200 °C ist;
wobei:
R¹ eine C₁-C₆-Alkylgruppe, eine C₆-C₁₂-Arylgruppe oder eine Heteroarylgruppe mit einem oder zwei Atomen, ausgewählt aus Stickstoff, Sauerstoff und Schwefel, ist;
R² Wasserstoff, eine C₁-C₆-Alkylgruppe, eine C₆-C₁₂-Arylgruppe, eine Heteroarylgruppe mit einem oder zwei Atomen, ausgewählt aus Stickstoff, Sauerstoff und Schwefel, eine C₁-C₆-Alkoxygruppe oder eine C₆-C₁₂-Aryloxygruppe ist;
R³ und R⁴ jeweils unabhängig Wasserstoff, eine C₁-C₆-Alkylgruppe, eine C₆-C₁₂-Arylgruppe oder eine Heteroarylgruppe mit einem oder zwei Atomen, ausgewählt aus Stickstoff, Sauerstoff und Schwefel, sind;
Y¹ und Y² jeweils unabhängig ein Halogen, eine C₁-C₆-Alkylsulfonyloxygruppe oder eine C₆-C₁₂-Arylsulfonyloxygruppe sind; und
m und n jeweils unabhängig 1 oder 2 sind.

2. Verfahren gemäß Anspruch 1, **dadurch gekennzeichnet, dass**:
R¹ eine C₁-C₃-Alkylgruppe ist;
R² ausgewählt ist aus einer C₁-C₃-Alkylgruppe, einer C₁-C₃-Alkoxygruppe und einer C₆-C₁₂-Arylgruppe;
R³ und R⁴ jeweils unabhängig Wasserstoff sind;
m und n jeweils unabhängig 2 sind;
in Schritt 1 die Base in der Cyclisierungsreaktion ausgewählt ist aus einem Alkalimetall- oder Erdalkalimetallhydroxid, Carbonat, Hydrogencarbonat, Alkoholat, Hydrid und Alkylat oder einem Gemisch davon;
in Schritt 1 ein Molverhältnis der Base zu Verbindung (1) 1,5-4,0:1 ist, ein Reaktionslösungsmittel ausgewählt ist aus einem aromatischen Kohlenwasserstoff, einem Ether, einem Amid oder einem Gemisch davon, und die Reaktionstemperatur -10-150 °C ist.

3. Verfahren gemäß Anspruch 2, **dadurch gekennzeichnet, dass**:
in Schritt 1 die Base in der Zyklisierungsreaktion Natriumhydrid ist, das Molverhältnis der Base zu Verbindung (1) 2,0-2,2:1 ist, das Reaktionslösungsmittel N,N-Dimethylformamid ist, und die Reaktionstemperatur 0-100 °C ist; und
in Schritt 2 die Base Kaliumhydroxid ist, das Molverhältnis von Base zu Verbindung (3) 3,4-4,5:1 ist, das Reaktionslösungsmittel Toluol oder Xylol ist, und die Reaktionstemperatur 100-130 °C ist.

4. Verfahren zum Herstellen einer oxadiazacyclischen Verbindung (4), umfassend:
Reagieren von Verbindung (3) mit einer Base, um eine oxadiazacyclische Verbindung (4) herzustellen, wie es in dem folgenden Reaktionsschema gezeigt ist: 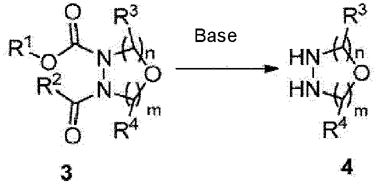 wobei:
R¹ eine C₁-C₆-Alkylgruppe, eine C₆-C₁₂-Arylgruppe oder eine Heteroarylgruppe mit einem oder zwei Atomen, ausgewählt aus Stickstoff, Sauerstoff und Schwefel, ist;
R² Wasserstoff, eine C₁-C₆-Alkylgruppe, eine C₆-C₁₂-Arylgruppe, eine Heteroarylgruppe mit einem oder zwei Atomen, ausgewählt aus Stickstoff, Sauerstoff und Schwefel, eine C₁-C₆-Alkoxygruppe oder eine C₆-C₁₂-Aryloxygruppe ist;
R³ und R⁴ jeweils unabhängig Wasserstoff, eine C₁-C₆-Alkylgruppe, eine C₆-C₁₂-Arylgruppe oder eine Heteroarylgruppe mit einem oder zwei Atomen, ausgewählt aus Stickstoff, Sauerstoff und Schwefel, sind; und
m und n jeweils unabhängig 1 oder 2 sind; wobei
die Base ausgewählt ist aus einem Alkalimetall oder Erdalkalimetallhydroxid, Carbonat und Hydrogencarbonat oder einem Gemisch davon;
ein molares Verhältnis der Base zu Verbindung (3) 3,0-4,5:1 ist; ein Reaktionslösungsmittel ein aromatischer Kohlenwasserstoff ist; und eine Reaktionstemperatur 0-200 °C ist.

5. Verfahren gemäß Anspruch 4, **dadurch gekennzeichnet, dass**: die Base Kaliumhydroxid ist;
das Molverhältnis der Base zu Verbindung (3) 3,4-4,5:1 ist;
das Reaktionslösungsmittel Toluol oder Xylol ist; und
die Reaktionstemperatur 100-130°C ist.

6. Verfahren gemäß einem der Ansprüche 1 bis 5, **dadurch gekennzeichnet, dass** jede Stufe in Anwesenheit eines Phasentransferkatalysators ausgeführt wird, wobei der Phasentransferkatalysator aus einem quaternären Ammoniumsalz, einem quaternären Phosphoniumsalz und einer cyclischen Kronenetherverbindung.

7. Verfahren gemäß Anspruch 6, **dadurch gekennzeichnet, dass** der Phasentransferkatalysator vorzugsweise Tetrabutylammoniumbromid ist.

8. Verbindung der Formel (3) 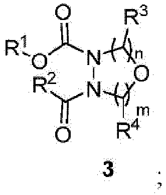 wobei:
R¹ eine C₁-C₆-Alkylgruppe oder eine C₆-C₁₂-Arylgruppe ist;
R² Wasserstoff, eine C₁-C₆-Alkylgruppe, eine C₆-C₁₂-Arylgruppe, eine Heteroarylgruppe mit einem oder zwei Atomen, ausgewählt aus Stickstoff, Sauerstoff und Schwefel, eine C₁-C₆-Alkoxygruppe oder eine C₆-C₁₂-Aryloxygruppe ist, und R² keine tert-Butoxygruppe ist, wenn R¹ eine tert-Butylgruppe ist;
R³ und R⁴ jeweils unabhängig Wasserstoff, eine C₁-C₆-Alkylgruppe, eine C₆-C₁₂-Arylgruppe oder eine Heteroarylgruppe mit einem oder zwei Atomen, ausgewählt aus Stickstoff, Sauerstoff und Schwefel, sind; und
m und n jeweils unabhängig 1 oder 2 sind.

9. Verbindung gemäß Anspruch 8, **dadurch gekennzeichnet, dass**:
R¹ eine C₁-C₃-Alkylgruppe ist;
R² eine C₁-C₃-Alkylgruppe, eine C₁-C₃-Alkoxygruppe oder eine Phenylgruppe ist;
R³ und R⁴ jeweils unabhängig Wasserstoff sind; und
m und n jeweils unabhängig 2 sind.

## Revendications

1. Un procédé pour préparer un composé oxadiazacyclique (4), comprenant :
étape 1 : la cyclisation du composé (1) avec le composé (2) en présence d'une base pour produire le composé (3) ; et
étape 2 : la réaction du composé (3) avec une base pour produire le composé oxadiazacyclique (4) ;
comme indiqué dans le schéma de réaction suivant : 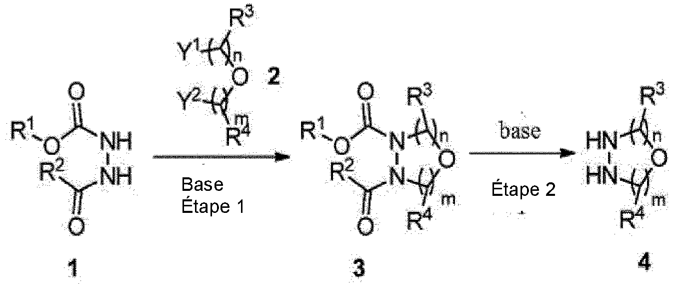 dans lequel, dans l'étape 2
la base est choisie parmi un hydroxyde de métal alcalin ou de métal alcalino-terreux, un carbonate et un hydrogénocarbonate ou un mélange de ceux-ci ; un rapport molaire de la base au composé (3) est de 3,0-4,5:1, un solvant de réaction est un hydrocarbure aromatique ; et une température de réaction est de 0-200 °C ;
dans lequel :
R¹ est un groupe alkyle en C₁-C₆, un groupe aryle en C₆-C₁₂, ou un groupe hétéroaryle contenant un ou deux atomes choisis parmi l'azote, l'oxygène et le soufre ;
R² est l'hydrogène, un groupe alkyle en C₁-C₆, un groupe aryle en C₆-C₁₂, un groupe hétéroaryle contenant un ou deux atomes choisis parmi l'azote, l'oxygène et le soufre, un groupe alcoxy en C₁-C₆, ou un groupe aryloxy en C₆-C₁₂ ;
R³ et R⁴ sont chacun indépendamment l'hydrogène, un groupe alkyle en C₁-C₆, un groupe aryle en C₆-C₁₂, ou un groupe hétéroaryle contenant un ou deux atomes choisis parmi l'azote, l'oxygène et le soufre ;
Y¹ et Y² sont chacun indépendamment un halogène, un groupe alkylsulfonyloxy en C₁-C₆, ou un groupe arylsulfonyloxy en C₆-C₁₂ ; et
m et n sont chacun indépendamment 1 ou 2.

2. Le procédé selon la revendication 1, **caractérisé en ce que** :
R¹ est un groupe alkyle en C₁-C₃ ;
R² est choisi parmi un groupe alkyle en C₁-C₃, un groupe alcoxy en C₁-C₃ et un groupe aryle en C₆-C₁₂ ;
R³ et R⁴ sont chacun indépendamment l'hydrogène ;
m et n sont chacun indépendamment 2 ;
dans l'étape 1, la base dans la réaction de cyclisation est choisie parmi un hydroxyde de métal alcalin ou de métal alcalino-terreux, un carbonate, un hydrogénocarbonate, un alcoolate, un hydrure et un alkylate, ou un mélange de ceux-ci ;
dans l'étape 1, le rapport molaire de la base au composé (1) est de 1,5-4,0:1, un solvant de réaction est choisi parmi un hydrocarbure aromatique, un éther, un amide, ou un mélange de ceux-ci, et une température de réaction est de - 10-150 °C.

3. Le procédé selon la revendication 2, **caractérisé en ce que** :
dans l'étape 1, la base dans la réaction de cyclisation est l'hydrure de sodium, le rapport molaire de la base au composé (1) est de 2,0-2,2:1, le solvant de réaction est le N,N-diméthylformamide, et la température de réaction est de 0-100 °C ; et
dans l'étape 2, la base est l'hydroxyde de potassium, le rapport molaire de la base au composé (3) est de 3,4-4,5:1, le solvant de réaction est le toluène ou le xylène, et la température de réaction est de 100-130 °C.

4. Un procédé pour préparer un composé oxadiazacyclique (4), comprenant :
la réaction du composé (3) avec une base pour produire le composé oxadiazacyclique (4), comme indiqué dans le schéma de réaction suivant : 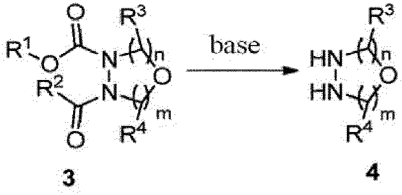 dans lequel :
R¹ est un groupe alkyle en C₁-C₆, un groupe aryle en C₆-C₁₂, ou un groupe hétéroaryle contenant un ou deux atomes choisis parmi l'azote, l'oxygène et le soufre ;
R² est l'hydrogène, un groupe alkyle en C₁-C₆, un groupe aryle en C₆-C₁₂, un groupe hétéroaryle contenant un ou deux atomes choisis parmi l'azote, l'oxygène et le soufre, un groupe alcoxy en C₁-C₆, ou un groupe aryloxy en C₆-C₁₂ ;
R³ et R⁴ sont chacun indépendamment l'hydrogène, un groupe alkyle en C₁-C₆, un groupe aryle en C₆-C₁₂, ou un groupe hétéroaryle contenant un ou deux atomes choisis parmi l'azote, l'oxygène et le soufre ; et
m et n sont chacun indépendamment 1 ou 2 ; dans lequel
la base est choisie parmi un hydroxyde de métal alcalin ou de métal alcalino-terreux, un carbonate et un hydrogénocarbonate, ou un mélange de ceux-ci ;
un rapport molaire de la base au composé (3) est de 3,0-4,5:1 ;
un solvant de réaction est un hydrocarbure aromatique ; et
une température de réaction est de 0-200 °C.

5. Le procédé selon la revendication 4, **caractérisé en ce que** :
la base est l'hydroxyde de potassium ;
le rapport molaire de la base au composé (3) est de 3,4-4,5:1 ;
le solvant de réaction est le toluène ou le xylène ; et
la température de réaction est de 100-130 °C.

6. Le procédé selon l'une quelconque des revendications 1 à 5, **caractérisé en ce que** chaque étape est effectuée en présence d'un catalyseur de transfert de phase, dans lequel le catalyseur de transfert de phase est choisi parmi un sel d'ammonium quaternaire, un sel de phosphonium quaternaire et un composé d'éther couronne cyclique.

7. Le procédé selon la revendication 6, **caractérisé en ce que** le catalyseur de transfert de phase est de préférence le bromure de tétrabutylammonium.

8. Un composé de formule (3) 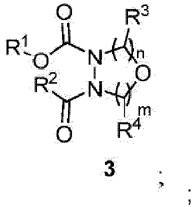 dans lequel :
R¹ est un groupe alkyle en C₁-C₆ ou un groupe aryle en C₆-C₁₂ ;
R² est l'hydrogène, un groupe alkyle en C₁-C₆, un groupe aryle en C₆-C₁₂, un groupe hétéroaryle contenant un ou deux atomes choisis parmi l'azote, l'oxygène et le soufre, un groupe alcoxy en C₁-C₆, ou un groupe aryloxy en C₆-C₁₂, et R² n'est pas un groupe tert-butoxy lorsque R¹ est un groupe tert-butyle ;
R³ et R⁴ sont chacun indépendamment l'hydrogène, un groupe alkyle en C₁-C₆, un groupe aryle en C₆-C₁₂, ou un groupe hétéroaryle contenant un ou deux atomes choisis parmi l'azote, l'oxygène et le soufre ; et
m et n sont chacun indépendamment 1 ou 2.

9. Le composé selon la revendication 8, **caractérisé en ce que** :
R¹ est un groupe alkyle en C₁-C₃ ;
R² est un groupe alkyle en C₁-C₃, un groupe alcoxy en C₁-C₃ ou un groupe phényle ;
R³ et R⁴ sont chacun indépendamment l'hydrogène ; et
m et n sont chacun indépendamment 2.
